# EUROPEAN PATENT APPLICATION

(11) **EP 3 257 381 A1**
(43) Date of publication of application: **20.12.2017**
(21) Application number: 16174759.7
(22) Date of filing: 16.06.2016
(51) Int. Cl.: A23K 20/189, A23K 50/75, A23K 50/30

(54) **USE OF BACTERIAL 3-PHYTASE FOR FEED OR FOOD PRODUCTS**

(71) Applicant: Clariant International Ltd, 4132 Muttenz (CH)
(72) Inventor: EISELE, Thomas, 82223 Eichenau (DE); HOESL, Michael, 81539 Muenchen (DE); KRAUS, Michael, 81241 München (DE); LEICHSENRING, Christoph, 81371 Muenchen (DE); LIST, Felix, 81476 Muenchen (DE); ROECHER, Lutz, 81541 Muenchen (DE); WALLRAPP, Frank, 82131 Gauting (DE)
(74) Representative: Graser, Konstanze

(57) **Abstract**

The present invention relates to the use of bacterial 3-phytases for the production of feed or food products. Further, the present invention relates to the use of bacterial 3-phytases for feeding animals and for the production of a food or feed additive.

SEQUENCE LISTING

<110> Clariant International Ltd

<120> Use of bacterial 3-phytase for feed or food products

<130> 2016DE604-EP-EPA

<160> 24

<170> BiSSAP 1.3.6

<210> 1
<211> 403
<212> PRT
<213> Dickeya zeae


<400> 1
Glu Thr Thr Ser Ser Gly Trp Thr Leu Glu Lys Val Val Glu Val Ser
1               5                   10                  15
Arg His Gly Val Arg Pro Pro Thr Glu Gly Asn Val Lys Thr Ile Gln
            20                  25                  30
Gln Gly Thr Asp Arg Gln Trp Pro Thr Trp Leu Thr His Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Lys Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asn Lys Lys Gly
            180                 185                 190
Asn Ile Ser Leu Arg Gly Pro Gly Ala Leu Ala Asn Met Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Gly
            260                 265                 270
His Gln Ala Asp Ala Pro Pro Asp Val Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg
                325                 330                 335
Ile Tyr Phe Gln Ala Gln Ser Leu Asp Gln Ile Arg Gly Leu Thr Ala
            340                 345                 350

Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val Arg
385                 390                 395                 400
Tyr Thr Pro

<210> 2
<211> 1209
<212> DNA
<213> Dickeya zeae

<400> 2
gaaaccacgt cgtcaggctg gacgctggaa aaagtggtgg aggtcagtcg ccacggtgtt      60

cgtccaccga ctgaaggtaa tgttaaaacc attcagcaag gcaccgatcg ccaatggccg     120

acctggctga cccattacgg cgaactgacc ggccacggct acgcggcagc ggtgttaaaa     180

gggcactacg aggggcagta cctgcgccag catgggttac tgagtcaggc ttgtccggct     240

cccggcgatg tgttcgtctg ggccagtccg ctacaacgca cacgggaaac cgcgatggcg     300

ctgatggacg gcgtgttccc cggttgtggt gtggcaattc aaggccctga ggatgaagac     360

aacgacccgc tgtttcatgc cgagacggca ggcgtgaagc tcgatcagca acaggtgaaa     420

gccgacctgc aaaacgcgat gcaggacaaa agcgccgcac aaattcaggc gcaatggcaa     480

ccggctatcg atcgtctgaa acaggcggtc tgcctgccgg ataaaccctg cccggcgttc     540

agcaccccct gggaagtgaa agagaataaa aaagggaata tttctctgcg cggccctggg     600

gcgctggcca atatggcgga gaccattcgg ctgtcctaca gcaacaataa tccactcagt     660

gaggtggcct tcggtcatgc taaaaccgcc gcagaagtgg cagcgctgat gccacttctg     720

accgccaatt acgacttcac caacgatctg ccctacgtgg ctcgccgcgg tgcgtcggta     780

ctgatgaacc agatagcgct ggcgctcaac accgggcatc aggctgatgc cccaccagat     840

gtcaaatggc tgctgtatgt ggcgcatgac accaacatcg ccaaactgcg tacgatgctg     900

ggctttacct ggcagatggg cgactatccg cgcggcaata tcccgcccgc aggtagcctt     960

atcttcgaac gctggcgcaa tcagcagtct ggtcagcgtg ttgtgcgtat ctatttccag    1020

gcacaatcgc tggatcagat tcgtgggctg actgcgctgg atgacagcca cccgccgtta    1080

cgcagcgagt tctcgatgcc tgactgtcag aaaaccgata tcggcaccct gtgcccgtat    1140

gacgcggtga tgaagcgcct taacgacgcg gtggaccgta ccgcgctgct gccagtgcgc    1200

tacacgccg                                                            1209

<210> 3
<211> 403
<212> PRT
<213> Dickeya dadantii

<400> 3
Asp Thr Pro Ser Ser Gly Trp Thr Leu Glu Lys Val Val Glu Val Ser
1               5                   10                  15
Arg His Gly Val Arg Pro Pro Thr Lys Gly Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Val Gln Gly Pro Glu Asp Glu Asp Asn Asp Pro Leu Phe His Ala Glu
        115                 120                 125
Ala Ala Gly Val Lys Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Ser Lys Lys Gly
            180                 185                 190
Asn Ile Ser Leu His Gly Pro Glu Ala Leu Ala Asn Ile Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Met Ala Arg Arg
                245                 250                 255
Gly Ala Ser Val Leu Met Asn Gln Ile Ala Leu Ala Leu Asn Thr Glu
            260                 265                 270
His Gln Ala Asp Val Pro Pro Asp Val Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Leu Val Arg
                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Ala Asp
        355                 360                 365
Cys Gln Lys Thr Asp Val Gly Thr Leu Cys Pro Tyr Asp Ala Val Met
    370                 375                 380
Lys Arg Leu Asn Asp Ala Val Asp Arg Thr Ala Leu Leu Pro Val Arg
385                 390                 395                 400
Tyr Thr Pro

<210> 4
<211> 1209
<212> DNA
<213> Dickeya dadantii

<400> 4

gacaccccgt cgtcaggctg gacgctggaa aaagtggtgg aagtcagtcg ccacggtgtc 60

cgcccgccga cgaaaggcaa tgttaaaacc attcaggaag gcaccgatcg ccagtggccg 120

acctggttga cgcagtacgg cgagctgacc ggtcacggct acgcggcagc agtgttaaaa 180

gggcactacg aggggcagta cctccgtcag catgggttac tgagtcaggc ttgtccggct 240

cccggcgacg tgttcgtctg ggccagtccg ctacaacgca cgcgagaaac tgcaatggcg 300

ctgatggatg gcgtgttccc cggctgtggt gtggcggtac aaggccctga ggatgaagac 360

aacgacccgc tgtttcatgc cgaggcggcg ggcgtgaagc tcgatcagca gcaggtgaaa 420

gccgacctgc aaaacgcgat gcaggacaaa agcgccgcgc aaattcaggc gcaatggcaa 480

ccggctatcg atcgtctgaa acaggcagtc tgcctgccgg ataaaccctg cccggcgttc 540

agtacgccct gggaagtgaa agagagtaaa aaagggaata tttctctgca cggccctgag 600

gcgctggcta acatcgcgga gactatccgg ctgtcctaca gcaacaataa tccgctcagc 660

gaggtggcct tcggtcatgc taaaaccgcc gcagaggtgg cagcgctgat gccacttctg 720

accgccaatt acgatttcac caacgatctg ccctacatgg cacgccgcgg tgcgtcggta 780

ctgatgaacc agatagcgct ggcgctaaac accgagcatc aggctgatgt cccaccagat 840

gtcaaatggc tactgtatgt ggcgcatgac accaacatcg ccaaactgcg tacgatgctg 900

ggctttacct ggcagatggg cgactatccg cgcggcaata tcccgcccgc aggtagcctt 960

atcttcgaac gctggcgcaa tcagcagtct ggtcagcggc tggtgcgtat ctatttccag 1020

gcacaatcgc tggatcagat tcgtggccta gcggcgctgg atgacagcca cccgccgtta 1080

cgcagcgagt tctcgatggc cgactgccag aaaaccgacg tcggcaccct gtgtccatat 1140

gacgcggtga tgaagcgcct taacgacgcg gtggaccgta ccgcgctgct gccagtgcgc 1200

tatacgccg 1209

<210> 5
<211> 403
<212> PRT
<213> Dickeya zeae

<400> 5
Glu Thr Thr Ser Ser Gly Trp Thr Leu Glu Lys Val Val Glu Val Ser
1               5                   10                  15
Arg His Gly Val Arg Pro Pro Thr Glu Gly Asn Val Lys Thr Ile Gln
            20                  25                  30
Gln Gly Thr Asp Arg Gln Trp Pro Ser Trp Leu Thr His Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Val Asp Gly Val Phe Pro Gly Cys Gly Val Ala

            100                 105                 110
Ile Gln Gly Pro Glu Asp Glu Asp Asn Asp Pro Leu Phe His Ala Glu
        115                 120                 125
Ala Ala Gly Val Lys Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Ser Lys Lys Gly
            180                 185                 190
Asn Ile Ser Leu His Gly Pro Glu Val Leu Ala Asn Ile Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Val Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg
                325                 330                 335
Ile Tyr Phe Gln Ala Gln Ser Leu Asp Gln Ile Arg Gly Leu Thr Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asn Ala Val Asp Arg Thr Ala Leu Leu Pro Val Arg
385                 390                 395                 400
Tyr Thr Pro

<210> 6
<211> 1209
<212> DNA
<213> Dickeya zeae

<400> 6
gaaaccacgt cgtcaggctg gacgctggaa aaagtggtgg aggtcagtcg ccacggtgtt 60

cgtccgccga ctgaaggcaa tgttaaaacc attcagcaag gcaccgatcg ccagtggcca 120

agctggctga cccattacgg cgaactgacc ggccacggct acgcagcagc ggtgttaaaa 180

gggcactacg aggggcagta cctgcgccag catgggttac tgagtcaggc ttgtccggct 240

cccggcgatg tgttcgtctg ggccagtccg ctgcaacgca cacgggaaac cgcgatggcg 300

ctagtggatg gcgtgttccc cggttgtggt gtggcgattc aaggccctga ggatgaagac 360

aacgacccgc tgtttcatgc cgaggcggca ggcgtgaagc tcgatcagca acaggtgaaa 420

gccgacctgc aaaacgcgat gcaggacaaa agcgccgcac aaattcaggc gcaatggcaa 480

ccggctatcg atcgcctgaa acaggcggtc tgtctgccgg ataaaccctg cccggcgttc 540

agcacgccct gggaagtgaa agagagtaaa aaagggaata tttctctgca cggccctgag 600

gtgctggcta acatcgcaga gactatccgg ctgtcctaca gtaacaataa tccgctcagt 660

gaggtggcct tcggtcatgc taaaactgcc gcagaggtgg cagcactgat gccgcttctg 720

accgccaatt acgatttcac caacgatctg ccctacgtgg cacgccgcgg tgcgtcggta 780

ctgatgaacc agatagcgct ggcactcaac accgagcatc aggctgatgc cccaccagat 840

gtcaaatggc tgctgtatgt ggcgcatgac accaacatcg ccaaactgcg tacgatgctc 900

ggctttacct ggcagatggg cgactatccg cgcggcaata tcccgcccgc aggtagcctt 960

atcttcgaac gctggcgcaa tcaacagtct ggtcagcgcg ttgtgcgtat ctatttccag 1020

gcacaatcgc tggatcagat tcgtgggctg actgcgctgg atgacagcca cccgccgtta 1080

cgcagcgagt tctcgatgcc tgactgtcag aaaaccgata tcggcaccct gtgcccgtat 1140

gacgcagtga tgaagcgcct taacaacgcg gtggaccgta ccgcgctact gccagtgcgc 1200

tatacgccg 1209

<210> 7
<211> 403
<212> PRT
<213> Dickeya zeae

<400> 7
Asp Thr Pro Ser Ser Gly Trp Thr Leu Glu Lys Val Val Glu Val Ser
1               5                   10                  15
Arg His Gly Val Arg Pro Pro Thr Lys Gly Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Ser Lys Lys Gly
            180                 185                 190
Asn Ile Ser Leu His Gly Pro Glu Val Leu Ala Asn Ile Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu

225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg
                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro

<210> 8
<211> 1209
<212> DNA
<213> Dickeya zeae

<400> 8
gacaccccgt cgtcaggctg gacgctggaa aaagtggtgg aagtcagtcg ccacggtgtc      60

cgcccgccga cgaaaggcaa tgttaaaacc attcaggaag gcaccgatcg ccagtggccg     120

acctggttga cgcagtacgg cgagctgacc ggtcacggct acgcggcagc ggtgttaaaa     180

gggcactacg aggggcagta cctgcgccag catgggttac tgagtcaggc ttgtccggct     240

cccggcgatg tgttcgtctg ggccagtccg ctacaacgca cgcgggaaac cgcaatggcg     300

ctgatggatg gcgtgttccc cggttgtggt gtggcgattc aaggccctga ggatgaagac     360

aacgacccgc tgtttcatgc cgagacggca ggtgtgacgc tcgatcagca gcaggtgaaa     420

gccgacctgc aaaacgcgat gcaggacaaa agcgccgcgc aaattcaggc gcaatggcaa     480

cccgctatcg atcgtctgaa acaggcagtc tgcctgccgg ataaaccctg cccggcgttc     540

agcacgccct gggaagtgaa agagagtaaa aaagggaata tttcactgca cggccctgag     600

gtgctggcca acatcgcgga gactatccgg ctgtcctaca gcaacaataa tccgctcagc     660

gaggtggcct tcggtcatgc taaaaccgcc gcagaggtgg ctgcgctgat gccacttctg     720

accgccaatt acgatttcac caacgatctg ccctacgtgg cacgccgcgg tgcgtcggta     780

ctgatgaacc agatagcgct ggcactcaac accgagcatc aggctgatgc cccaccagat     840

gccaaatggc tgctgtatgt ggcgcatgac accaacatcg ccaaactgcg tacgatgctg     900

ggctttacct ggcagatggg cgactatccg cgcggcaata tcccgcccgc aggtagcctt     960

atcttcgaac gctggcgcaa tcagcagtct ggtcagcgtg ttgtgcgtat ctatttccag 1020

gcacaatcgc tggatcagat tcgtgggctc gcggcgctgg atgacagcca cccgccgtta 1080

cgcagcgagt tctcgatgcc tgactgtcag aaaaccgata tcggtaccct gtgcccgtat 1140

gacgcggtga tgaagcgcct taacgacgcg gtggaccgta ccgcgctgct gccagtgcac 1200

tatacgccg 1209

<210> 9
<211> 403
<212> PRT
<213> Dickeya zeae

<400> 9
Glu Thr Thr Trp Ser Gly Trp Thr Leu Glu Lys Val Val Glu Val Ser
1               5                   10                  15
Arg His Gly Val Arg Pro Pro Thr Glu Gly Asn Val Lys Thr Ile Gln
            20                  25                  30
Gln Gly Thr Asp Arg Gln Trp Pro Ser Trp Leu Thr His Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Val Asp Gly Val Phe Pro Gly Cys Gly Val Ala
            100                 105                 110
Val Arg Gly Pro Glu Asp Glu Asp Asn Asp Pro Leu Phe His Ala Glu
        115                 120                 125
Ala Ala Gly Val Lys Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asn Lys Lys Gly
            180                 185                 190
Asn Ile Ser Leu Arg Gly Pro Gly Ala Leu Ala Asn Met Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Met Ala Arg Arg
                245                 250                 255
Gly Ala Ser Val Leu Met Asn Gln Ile Ala Leu Ala Leu Asn Thr Gly
            260                 265                 270
His Gln Ala Asp Val Pro Pro Asp Val Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Leu Val Arg
                325                 330                 335
Ile Tyr Phe Gln Ala Gln Ser Leu Asp Gln Ile Arg Gly Leu Thr Ala
            340                 345                 350
Leu Asp Asp Ser His Pro Pro Leu Arg Ser Glu Phe Ser Met Ala Asp

355                 360                 365
Cys Gln Lys Thr Asp Val Gly Thr Leu Cys Pro Tyr Asp Ala Val Met
    370                 375                 380
Lys Arg Leu Asn Asn Ala Val Asp Arg Ser Ala Leu Leu Pro Val His
385                 390                 395                 400
Tyr Thr Pro

<210> 10
<211> 1209
<212> DNA
<213> Artificial Sequence

<220>
<223> -

<400> 10
gagaccacct ggtccggctg gaccctcgag aaggtcgtcg aggtctcccg ccacggcgtc      60

cgccccccca ccgagggcaa cgtcaagacc atccagcagg gcaccgaccg ccagtggccc     120

tcctggctca cccactacgg cgagctcacc ggccacggct acgccgccgc cgtcctcaag     180

ggccactacg agggccagta cctccgccag cacggcctcc tctcccaggc ctgccccgcc     240

cccggcgacg tcttcgtctg ggcctccccc ctccagcgca cccgcgagac cgccatggcc     300

ctcgtcgacg gcgtcttccc cggctgcggc gtcgccgtcc gcggccccga ggacgaggac     360

aacgaccccc tcttccacgc cgaggccgcc ggcgtcaagc tcgaccagca gcaggtcaag     420

gccgacctcc agaacgccat gcaggacaag tccgccgccc agatccaggc ccagtggcag     480

cccgccatcg accgcctcaa gcaggccgtc tgcctccccg acaagccctg ccccgccttc     540

tccaccccct gggaggtcaa ggagaacaag aagggcaaca tctccctccg cggccccggc     600

gccctcgcca acatggccga gaccatccgc ctctcctact ccaacaacaa ccccctctcc     660

gaggtcgcct tcggccacgc caagaccgcc gccgaggtcg ccgccctcat gcccctcctc     720

accgccaact acgacttcac caacgacctc ccctacatgg cccgccgcgg cgcctccgtc     780

ctcatgaacc agatcgccct cgccctcaac accggccacc aggccgacgt cccccccgac     840

gtcaagtggc tcctctacgt cgcccacgac accaacatcg ccaagctccg caccatgctc     900

ggcttcacct ggcagatggg cgactacccc cgcggcaaca tcccccccgc cggctccctc     960

atcttcgagc gctggcgcaa ccagcagtcc ggccagcgcc tcgtccgcat ctacttccag    1020

gcccagtccc tcgaccagat ccgcggcctc accgccctcg acgactccca cccccccctc    1080

cgctccgagt tctccatggc cgactgccag aagaccgacg tcggcaccct ctgcccctac    1140

gacgccgtca tgaagcgcct caacaacgcc gtcgaccgct ccgccctcct ccccgtccac    1200

tacaccccc                                                            1209

<210> 11
<211> 404

<212> PRT
<213> Dickeya chrysanthemi

<400> 11
   Glu Thr Thr Pro Ser Gly Trp Thr Leu Glu Lys Val Val Glu Val Ser
   1               5                   10                  15
   Arg His Gly Val Arg Pro Pro Thr Glu Gly Asn Val Lys Thr Ile Gln
               20                  25                  30
   Glu Gly Thr Gly Arg Asn Trp Pro Thr Trp Leu Thr His Tyr Gly Glu
           35                  40                  45
   Leu Thr Gly His Gly Tyr Ala Ala Ala Val Leu Lys Gly Gln Tyr Glu
       50                  55                  60
   Gly Gln Tyr Leu Arg Gln His Gly Leu Leu Ser Gln Asn Cys Pro Thr
   65                  70                  75                  80
   Pro Gly Asp Val Phe Val Trp Ala Ser Pro Leu Gln Arg Thr Gln Glu
                   85                  90                  95
   Thr Ala Met Ala Leu Met Asp Gly Val Phe Pro Gly Cys Gly Val Val
               100                 105                 110
   Ile Arg Gly Pro Glu Ser Glu Asp Asn Asp Thr Leu Phe His Ala Glu
           115                 120                 125
   Thr Ala Gly Val Gln Met Asp Gln Glu Gln Val Lys Ala Ser Leu Gln
       130                 135                 140
   Asn Ala Met Gln Gly Lys Ser Ala Glu Gln Ile Gln Ala Glu Trp Gln
   145                 150                 155                 160
   Pro Ala Ile Asp Arg Leu Lys Gln Ala Val Cys Leu Pro Asp Thr Ala
                   165                 170                 175
   Cys Pro Ala Phe Ser Ala Lys Trp Glu Val Lys Glu Ser Lys Lys Gly
               180                 185                 190
   Asn Ile Ser Leu His Gly Pro Glu Ala Leu Ala Asn Met Ala Glu Thr
           195                 200                 205
   Ile Arg Leu Ser Tyr Ser Asn Asn Asn Pro Leu Gly Gln Val Ala Phe
       210                 215                 220
   Gly Asn Ala Gly Thr Ala Ala Ala Val Ala Ala Leu Met Pro Leu Leu
   225                 230                 235                 240
   Thr Ala Asn Tyr Asp Phe Thr Asn Asp Leu Pro Tyr Val Ala Arg Arg
                   245                 250                 255
   Gly Gly Ser Ile Leu Met Asn Gln Ile Ala Leu Ala Leu Asn Pro Glu
               260                 265                 270
   Arg His Gln Glu Asp Ala Pro Pro Asp Ala Lys Trp Leu Leu Tyr Val
           275                 280                 285
   Ala His Asp Thr Asn Ile Ala Lys Leu Arg Ala Met Leu Gly Phe Thr
       290                 295                 300
   Trp Gln Met Gly Asp Tyr Pro Arg Gly Asn Ile Pro Pro Ala Gly Ser
   305                 310                 315                 320
   Leu Ile Phe Glu Arg Trp Arg Asn Ala Gln Ser Gly Gln Arg Phe Val
                   325                 330                 335
   Arg Ile Tyr Phe Gln Ala Gln Ser Leu Asp Gln Ile Arg Gly Leu Thr
               340                 345                 350
   Thr Leu Asp Asn Asp His Pro Pro Leu Arg Ser Glu Phe Ser Ile Asn
           355                 360                 365
   Gly Cys Gln Lys Ile Asp Val Gly Thr Leu Cys Pro Tyr Asp Ala Val
       370                 375                 380
   Met Lys Arg Leu Asn Glu Ser Val Asp Arg Thr Ala Leu Leu Pro Val
   385                 390                 395                 400
   Arg Tyr Ala Leu

<210> 12
<211> 1212
<212> DNA
<213> Artificial Sequence

<220>
<223> -

<400> 12
gaaaccaccc cgtcgggctg gacgctggaa aaagtcgtgg aagtcagccg tcatggcgtt      60

cgcccgccga ccgaaggcaa cgttaaaacc attcaggaag gcaccggccg caactggccg     120

acctggctga cccattacgg cgaactgacc ggccacggct acgcggcggc cgtgctaaaa     180

ggccagtacg aagggcagta cctgcgtcag cacgggttgc ttagtcagaa ttgcccgacg     240

cccggcgacg tgtttgtctg ggccagcccg ctgcaacgca cgcaggaaac cgccatggcg     300

ctgatggatg gtgtattccc cggttgcggc gtggtgattc gcggcccgga gagtgaagac     360

aacgacacat tgttccatgc cgagacggcc ggcgtgcaga tggatcagga acaggtgaaa     420

gcctctttgc aaaacgcgat gcagggtaaa agcgccgaac aaattcaggc ggagtggcaa     480

ccggccatcg atcgcctgaa acaggcggtc tgcctgccgg ataccgcttg cccggcgttc     540

agcgccaaat gggaagtgaa agaaagtaag aaagggaata tctcgctgca cggcccggaa     600

gcgctggcca atatggcgga aaccatccgc ttgtcctaca gcaataacaa cccgctcggc     660

caggtggcct tcggcaatgc cgggaccgct gccgcggtgg cggcgctgat gccgctgctg     720

accgccaact acgatttcac caacgacctg ccctatgtgg cacggcgcgg cggttcgata     780

ctgatgaacc agatagccct ggcgcttaac ccggaacgac atcaggagga tgccccaccc     840

gatgccaaat ggctgctgta cgtggcgcac gataccaaca tcgcgaaact gcgtgccatg     900

ctgggcttca cctggcagat gggcgactac ccgcgcggca atatcccgcc cgccggcagc     960

ctgatcttcg agcgctggcg caacgcgcag tccggtcaac gcttcgtgcg catctacttt    1020

caggcgcaat cgctggacca gattcgcggg ctgaccacgt tggataacga ccatccgccg    1080

ctgcgcagcg aattctcaat caacggttgc cagaaaatcg atgtcggcac cctgtgtccg    1140

tacgatgccg tgatgaagcg cctcaacgaa tcggtggatc gcaccgcgct gctgccggtg    1200

cgctacgcgc tg                                                        1212

<210> 13
<211> 403
<212> PRT
<213> Dickeya dadantii

<400> 13
Glu Thr Ala Pro Ser Gly Trp Ala Leu Glu Lys Val Val Glu Ile Ser
1               5                   10                  15
Arg His Gly Val Arg Pro Pro Thr Glu Gly Asn Val Lys Thr Ile Gln
            20                  25                  30
Glu Gly Thr Gly Arg Asp Trp Pro Thr Trp Leu Thr His Tyr Gly Glu
        35                  40                  45
Leu Thr Gly His Gly Tyr Ala Ala Ala Val Leu Lys Gly Gln Tyr Glu
    50                  55                  60

Gly Gln Tyr Leu Arg Gln His Gly Leu Leu Thr Arg Asn Cys Pro Ala
65                  70                  75                  80
Pro Asp Asp Val Phe Val Trp Ala Ser Pro Leu Gln Arg Thr Gln Glu
                85                  90                  95
Thr Ala Met Ala Leu Met Asp Gly Val Phe Pro Gly Cys Gly Val Val
            100                 105                 110
Ile Arg Gly Pro Glu Ser Glu Asp Asn Asp Pro Leu Phe His Ala Glu
        115                 120                 125
Thr Ala Gly Val Leu Val Asp Gln Glu Gln Val Lys Val Asp Leu Gln
    130                 135                 140
Lys Ala Met Gln Gly Lys Ser Ala Glu Gln Ile Gln Ala Glu Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Ala Lys Trp Glu Leu Lys Glu Ser Lys Lys Gly
            180                 185                 190
Asn Thr Ser Leu His Gly Pro Glu Ala Leu Ala Asn Met Ala Glu Thr
        195                 200                 205
Ile Arg Leu Ala Tyr Ser Asn Asn Asn Pro Leu Gly Gln Val Ala Phe
    210                 215                 220
Gly Asn Ala Arg Thr Ala Ala Ala Val Gly Ser Leu Met Ser Leu Leu
225                 230                 235                 240
Thr Ala Asn Tyr Asp Phe Thr Asn Asp Leu Pro Tyr Val Ala Arg Arg
                245                 250                 255
Gly Gly Ser Val Leu Met Asn Gln Ile Ala Leu Ala Leu Asn Pro Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Ala Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Ala Gln Ser Gly Gln Arg Phe Val Arg
                325                 330                 335
Ile Tyr Phe Gln Ala Gln Ser Leu Asp Gln Ile Arg Gly Leu Thr Ala
            340                 345                 350
Leu Asp Asn Asp His Pro Pro Leu Arg Ser Glu Phe Ser Met Asn Gly
        355                 360                 365
Cys Gln Lys Thr Asp Val Gly Thr Leu Cys Pro Tyr Asp Ala Val Met
    370                 375                 380
Lys His Leu Asn Gly Ala Val Asp Arg Thr Ala Leu Leu Pro Val Arg
385                 390                 395                 400
Tyr Ser Leu

<210> 14
<211> 1209
<212> DNA
<213> Dickeya dadantii

<400> 14
gaaaccgctc cgtcaggctg ggcactggaa aaagtggtgg aaataagccg ccacggcgta      60

cgcccgccga ccgagggcaa cgtcaaaacc attcaggaag gcaccgggcg cgactggccg     120

acctggctga cccattacgg cgagctgacc ggtcacggtt atgcggcggc ggtactaaaa     180

gggcaatacg aagggcagta cctgcgccag cacggactgc tgacccgcaa ttgcccggcg     240

cccgacgacg tatttgtctg ggccagcccg ctgcaacgca cgcaggaaac cgccatggcg     300

ttgatggatg gcgtatttcc tggctgcggc gtcgtgattc gcggcccgga gagtgaagac     360

aacgacccgc tgttccatgc cgaaaccgcc ggcgtgctgg tggatcagga acaggtgaag 420

gtcgacctgc aaaaagcgat gcagggcaaa agcgccgaac agattcaggc ggagtggcaa 480

ccggcgatcg atcgcctgaa acaggcggtc tgcctgccgg ataaaccctg cccggcgttc 540

agcgccaaat gggaattgaa agagagcaaa aaaaggaata cctcgctgca cggcccggag 600

gcgctggcca acatggcgga aaccatccgg ctggcctaca gcaacaacaa cccgctcggt 660

caggtggcgt tcggcaacgc cagaacggcc gccgcggtgg gttcgctaat gtcgttgctg 720

accgccaact acgatttcac caacgacctg ccctacgtgg cgcggcgcgg cggttcggta 780

ctgatgaatc agatagccct ggcgcttaac ccggaacatc aggcggacgc cccgcccgac 840

gccaaatggc tgctgtacgt ggcgcacgat accaacatcg ccaaattgcg cgccatgctg 900

ggtttcacct ggcagatggg cgactacccg cgcggtaata tcccgcccgc gggcagcctg 960

attttcgaac gctggcgcaa cgcgcagtcc ggtcaacgct tcgtgcgcat ctacttccag 1020

gcgcagtcgc tggaccagat tcgcgggctg accgcgctgg ataacgacca tccgccgctg 1080

cgcagcgagt tctcgatgaa cggctgccag aaaaccgatg tcggcaccct gtgcccatac 1140

gatgcggtga tgaagcacct caacggcgcg gtagaccgca ccgcgctgct gccggtgcgc 1200

tactcgctg 1209

<210> 15
<211> 402
<212> PRT
<213> Dickeya paradisiaca

<400> 15
Ala Ser Thr Ser Trp Ser Leu Glu Lys Val Val Glu Ile Ser Arg His
1               5                   10                  15
Gly Val Arg Pro Pro Thr Glu Gly Asn Ile Lys Thr Ile Gln Glu Gly
            20                  25                  30
Thr Gly Arg Glu Trp Pro Thr Trp Leu Thr Arg Tyr Gly Glu Leu Thr
        35                  40                  45
Gly His Gly Tyr Ala Ala Ala Val Leu Lys Gly Gln Tyr Glu Gly Ser
    50                  55                  60
Tyr Leu Arg Glu Asn Ala Leu Leu Thr Gly Ala Cys Pro Ala Ser Gly
65                  70                  75                  80
Glu Val Phe Val Trp Ala Ser Pro Leu Gln Arg Thr Gln Glu Thr Ala
                85                  90                  95
Met Ala Leu Met Asp Gly Val Phe Pro Gly Cys Gly Ile Thr Ile Arg
            100                 105                 110
Gly Ala Ala Ser Glu Glu Gln Asp Thr Leu Phe His Ala Asp Asp Ala
        115                 120                 125
Gly Val Thr Leu Asp Ala Glu Gln Val Arg Ala Asp Leu Gln Lys Ala
    130                 135                 140
Met Gln Asn Lys Thr Ala Ala Gln Leu Gln Thr Gly Phe Lys Pro Asp
145                 150                 155                 160
Ile Glu Arg Leu Gln Arg Ala Val Cys Gln Thr Asp Asn Lys Pro Cys
                165                 170                 175
Pro Ala Phe Ser Ala Gln Trp Asp Val Lys Asp Gly Lys Lys Gly Tyr
            180                 185                 190

Pro Val Leu Asn Gly Pro Ala Ala Leu Ala Asn Met Ala Glu Thr Ile
        195                 200                 205
Arg Leu Ala Tyr Ser Asn Asn Ala Pro Leu Ser Gln Val Ala Phe Gly
    210                 215                 220
Asn Ala Arg Ser Ala Val Asp Val Gly Ala Leu Met Ser Leu Leu Thr
225                 230                 235                 240
Val Asn Tyr Asp Phe Thr Asn Asp Leu Pro Tyr Val Ala Arg Arg Gly
                245                 250                 255
Ala Ser Asn Val Leu Asn Gln Ile Ala Leu Ser Leu Ser Thr Gln Pro
            260                 265                 270
Gln Pro Asp Ala Pro Pro Ala Ala Lys Trp Leu Leu Phe Val Ala His
        275                 280                 285
Asp Thr Asn Ile Ala Gln Leu Arg Thr Leu Leu Gly Phe Thr Trp Lys
    290                 295                 300
Gln Ala Glu Tyr Pro Arg Gly Asn Ile Pro Pro Ala Gly Ser Leu Ile
305                 310                 315                 320
Phe Glu Arg Trp Arg Asn Asn Gln Ser Gly Glu Arg Phe Leu Arg Ile
                325                 330                 335
Tyr Phe Gln Ala Gln Ser Leu Asp Gln Ile Arg Ala Leu Met Pro Leu
            340                 345                 350
Asp Ser Gly Asn Pro Pro Leu Arg Ser Glu Phe Thr Thr Asp Gly Cys
        355                 360                 365
Gln Gln Thr Glu Val Gly Thr Leu Cys Pro Tyr Asp Ala Ala Leu Gln
    370                 375                 380
Arg Leu Asn Asp Ala Ile Asp Arg Thr Ala Leu Leu Pro Val Ser Tyr
385                 390                 395                 400
Ser Arg

<210> 16
<211> 1206
<212> DNA
<213> Dickeya paradisiaca

<400> 16
gcctccacat catggagcct ggaaaaagtg gtggaaatca gccgccacgg tgtacgcccg      60

ccgacggaag gcaatatcaa aaccattcag gaaggtaccg gtcgggaatg gccgacctgg     120

ctgacccgct atggcgagtt gaccggtcac ggctacgctg ccgcagtgct gaaaggccag     180

tatgaaggca gttatctgcg cgaaaatgcg ctgctgaccg gcgcttgccc agcctccggc     240

gaagtatttg tctgggccag cccgttgcaa cgcacgcagg aaaccgccat ggcgctgatg     300

gacggtgtct tccccggctg cggcatcact atccgcggcg cggcaagcga agaacaggac     360

acgttgttcc acgccgacga cgccggtgtc acgctggatg ccgagcaggt tcgcgccgat     420

ttgcaaaaag cgatgcagaa caaaaccgca gcccagcttc agaccggttt caaaccggat     480

atcgaacgcc tgcaacgcgc ggtttgccag accgacaata aaccctgccc ggcctttagc     540

gcccaatggg acgttaaaga cggcaagaaa ggttatccgg tgctgaatgg cccggcggcg     600

ttggccaaca tggcggaaac catccgtctg gcgtacagca acaacgcgcc gctcagccag     660

gtagctttcg gcaatgcccg cagtgcggtc gacgtcggcg cgctgatgtc gctgctgacc     720

gtcaattatg atttcaccaa cgatctgccc tatgtcgccc gtcgcggcgc ctccaatgtg     780

ctcaaccaga tcgcgctctc actgtcgaca caaccgcagc cggacgcccc gcccgccgcc     840

aaatggctgt tgttcgtggc gcacgacacc aatatcgccc agttacgcac cctgttgggg 900

ttcacctgga aacaggcgga atatccgcgc ggcaatattc cgccggcagg cagcctgatt 960

ttcgaacgtt ggcgcaacaa tcagtctggc gagcgttttt tacgcatcta tttccaggcg 1020

caatcgctgg atcagatccg ggcgttgatg ccgctggata gcggcaaccc gccgttacgc 1080

agcgaattca ccaccgatgg atgtcagcaa accgaagtgg ggactctgtg cccatacgac 1140

gcggccctgc aacgtcttaa tgacgctatc gatcgcaccg cgctgctgcc ggtaagctat 1200

agtcgc 1206

<210> 17
<211> 411
<212> PRT
<213> Escherichia coli

<400> 17
Met Gln Ser Glu Pro Glu Leu Lys Leu Glu Ser Val Val Ile Val Ser
1               5                   10                  15
Arg His Gly Val Arg Ala Pro Thr Lys Ala Thr Gln Leu Met Gln Asp
            20                  25                  30
Val Thr Pro Asp Ala Trp Pro Thr Trp Pro Val Lys Leu Gly Trp Leu
        35                  40                  45
Thr Pro Arg Gly Gly Glu Leu Ile Ala Tyr Leu Gly His Tyr Gln Arg
    50                  55                  60
Gln Arg Leu Val Ala Asp Gly Leu Leu Ala Lys Lys Gly Cys Pro Gln
65                  70                  75                  80
Ser Gly Gln Val Ala Ile Ile Ala Asp Val Asp Glu Arg Thr Arg Lys
                85                  90                  95
Thr Gly Glu Ala Phe Ala Ala Gly Leu Ala Pro Asp Cys Ala Ile Thr
            100                 105                 110
Val His Thr Gln Ala Asp Thr Ser Ser Pro Asp Pro Leu Phe Asn Pro
        115                 120                 125
Leu Lys Thr Gly Val Cys Gln Leu Asp Asn Ala Asn Val Thr Asp Ala
    130                 135                 140
Ile Leu Ser Arg Ala Gly Gly Ser Ile Ala Asp Phe Thr Gly His Arg
145                 150                 155                 160
Gln Thr Ala Phe Arg Glu Leu Glu Arg Val Leu Asn Phe Pro Gln Ser
                165                 170                 175
Asn Leu Cys Leu Lys Arg Glu Lys Gln Asp Glu Ser Cys Ser Leu Thr
            180                 185                 190
Gln Ala Leu Pro Ser Glu Leu Lys Val Ser Ala Asp Asn Val Ser Leu
        195                 200                 205
Thr Gly Ala Val Ser Leu Ala Ser Met Leu Thr Glu Ile Phe Leu Leu
    210                 215                 220
Gln Gln Ala Gln Gly Met Pro Glu Pro Gly Trp Gly Arg Ile Thr Asp
225                 230                 235                 240
Ser His Gln Trp Asn Thr Leu Leu Ser Leu His Asn Ala Gln Phe Tyr
                245                 250                 255
Leu Leu Gln Arg Thr Pro Glu Val Ala Arg Ser Arg Ala Thr Pro Leu
            260                 265                 270
Leu Asp Leu Ile Lys Thr Ala Leu Thr Pro His Pro Pro Gln Lys Gln
        275                 280                 285
Ala Tyr Gly Val Thr Leu Pro Thr Ser Val Leu Phe Ile Ala Gly His
    290                 295                 300
Asp Thr Asn Leu Ala Asn Leu Gly Gly Ala Leu Glu Leu Asn Trp Thr
305                 310                 315                 320

Leu Pro Gly Gln Pro Asp Asn Thr Pro Pro Gly Gly Glu Leu Val Phe
                325                 330                 335
Glu Arg Trp Arg Arg Leu Ser Asp Asn Ser Gln Trp Ile Gln Val Ser
            340                 345                 350
Leu Val Phe Gln Thr Leu Gln Gln Met Arg Asp Lys Thr Pro Leu Ser
        355                 360                 365
Leu Asn Thr Pro Pro Gly Glu Val Lys Leu Thr Leu Ala Gly Cys Glu
    370                 375                 380
Glu Arg Asn Ala Gln Gly Met Cys Ser Leu Ala Gly Phe Thr Gln Ile
385                 390                 395                 400
Val Asn Glu Ala Arg Ile Pro Ala Cys Ser Leu
                405                 410

<210> 18
<211> 1296
<212> DNA
<213> Escherichia coli


<400> 18
atgaaagcga tcttaatccc atttttatct cttctgattc cgttaacccc gcaatctgca      60

ttcgctcaga gtgagccgga gctgaagctg gaaagtgtgg tgattgtcag tcgtcatggt     120

gtgcgtgctc caaccaaggc cacgcaactg atgcaggatg tcaccccaga cgcatggcca     180

acctggccgg taaaactggg ttggctgaca ccgcgcggtg gtgagctaat cgcctatctc     240

ggacattacc aacgccagcg tctggtagcc gacggattgc tggcgaaaaa gggctgcccg     300

cagtctggtc aggtcgcgat tattgctgat gtcgacgagc gtacccgtaa aacaggcgaa     360

gccttcgccg ccgggctggc acctgactgt gcaataaccg tacataccca ggcagatacg     420

tccagtcccg atccgttatt taatcctcta aaaactggcg tttgccaact ggataacgcg     480

aacgtgactg acgcgatcct cagcagggca ggagggtcaa ttgctgactt taccgggcat     540

cggcaaacgg cgtttcgcga actggaacgg gtgcttaatt ttccgcaatc aaacttgtgc     600

cttaaacgtg agaaacagga cgaaagctgt tcattaacgc aggcattacc atcggaactc     660

aaggtgagcg ccgacaatgt ctcattaacc ggtgcggtaa gcctcgcatc aatgctgacg     720

gagatatttc tcctgcaaca agcacaggga atgccggagc cggggtgggg aaggatcacc     780

gattcacacc agtggaacac cttgctaagt ttgcataacg cgcaatttta tttgctacaa     840

cgcacgccag aggttgcccg cagccgcgcc accccgttat tagatttgat caagacagcg     900

ttgacgcccc atccaccgca aaaacaggcg tatggtgtga cattacccac ttcagtgctg     960

tttatcgccg gacacgatac taatctggca aatctcggcg gcgcactgga gctcaactgg    1020

acgcttcccg gtcagccgga taacacgccg ccaggtggtg aactggtgtt tgaacgctgg    1080

cgtcggctaa gcgataacag ccagtggatt caggtttcgc tggtcttcca gactttacag    1140

cagatgcgtg ataaaacgcc gctgtcatta aatacgccgc ccggagaggt gaaactgacc    1200

ctggcaggat gtgaagagcg aaatgcgcag ggcatgtgtt cgttggcagg ttttacgcaa    1260

atcgtgaatg aagcacgcat accggcgtgc agtttg                              1296

<210> 19
<211> 412
<212> PRT
<213> Citrobacter braakii

<400> 19
Met Glu Glu Gln Asn Gly Met Lys Leu Glu Arg Val Val Ile Val Ser
1               5                   10                  15
Arg His Gly Val Arg Ala Pro Thr Lys Phe Thr Pro Ile Met Lys Asp
            20                  25                  30
Val Thr Pro Asp Gln Trp Pro Gln Trp Asp Val Pro Leu Gly Trp Leu
        35                  40                  45
Thr Pro Arg Gly Gly Glu Leu Val Ser Glu Leu Gly Gln Tyr Gln Arg
    50                  55                  60
Leu Trp Phe Thr Ser Lys Gly Leu Leu Asn Asn Gln Thr Cys Pro Ser
65                  70                  75                  80
Pro Gly Gln Val Ala Val Ile Ala Asp Thr Asp Gln Arg Thr Arg Lys
                85                  90                  95
Thr Gly Glu Ala Phe Leu Ala Gly Leu Ala Pro Lys Cys Gln Ile Gln
            100                 105                 110
Val His Tyr Gln Lys Asp Glu Glu Lys Ala Asp Pro Leu Phe Asn Pro
        115                 120                 125
Val Lys Met Gly Lys Cys Ser Phe Asn Thr Leu Lys Val Lys Asn Ala
    130                 135                 140
Ile Leu Glu Arg Ala Gly Gly Asn Ile Glu Leu Tyr Thr Gln Arg Tyr
145                 150                 155                 160
Gln Ser Ser Phe Arg Thr Leu Glu Asn Val Leu Asn Phe Ser Gln Ser
                165                 170                 175
Glu Thr Cys Lys Thr Thr Glu Lys Ser Thr Lys Cys Thr Leu Pro Glu
            180                 185                 190
Ala Leu Pro Ser Glu Leu Lys Val Thr Pro Asp Asn Val Ser Leu Pro
        195                 200                 205
Gly Ala Trp Ser Leu Ser Ser Thr Leu Thr Glu Ile Phe Leu Leu Gln
    210                 215                 220
Glu Ala Gln Gly Met Pro Gln Val Ala Trp Gly Arg Ile Thr Gly Glu
225                 230                 235                 240
Lys Glu Trp Arg Asp Leu Leu Ser Leu His Asn Ala Gln Phe Asp Leu
                245                 250                 255
Leu Gln Arg Thr Pro Glu Val Ala Arg Ser Arg Ala Thr Pro Leu Leu
            260                 265                 270
Asp Met Ile Asp Thr Thr Leu Leu Thr Asn Gly Ala Thr Glu Asn Arg
        275                 280                 285
Tyr Gly Ile Lys Leu Pro Val Ser Leu Leu Phe Ile Ala Gly His Asp
    290                 295                 300
Thr Asn Leu Ala Asn Leu Ser Gly Ala Leu Asp Leu Asn Trp Ser Leu
305                 310                 315                 320
Pro Gly Gln Pro Asp Asn Thr Pro Pro Gly Gly Glu Leu Val Phe Glu
                325                 330                 335
Lys Trp Lys Arg Thr Ser Asp Asn Thr Asp Trp Val Gln Val Ser Phe
            340                 345                 350
Val Tyr Gln Thr Leu Arg Asp Met Arg Asp Ile Gln Pro Leu Ser Leu
        355                 360                 365
Glu Lys Pro Ala Gly Lys Val Asp Leu Lys Leu Ile Ala Cys Glu Glu
    370                 375                 380
Lys Asn Ile Gln Gly Met Cys Ser Leu Lys Ser Phe Ser Arg Leu Ile
385                 390                 395                 400
Lys Glu Ile Arg Val Pro Glu Cys Ala Val Thr Glu
                405                 410

<210> 20

<211> 1236
<212> DNA
<213> Citrobacter braakii

<400> 20
atggaagagc agaacggcat gaaacttgag cgggttgtga tagtgagtcg tcatggagta 60

agagcaccta cgaagttcac tccaataatg aaagatgtca cacccgatca atggccacaa 120

tgggatgtgc cgttaggatg gctaacgcct cgtgggggag aacttgtttc tgaattaggt 180

cagtatcaac gtttatggtt cacgagcaaa ggtctgttga ataatcaaac gtgcccatct 240

ccagggcagg ttgctgttat tgcagacacg gatcaacgca cccgtaaaac gggtgaggcg 300

tttctggctg ggttagcacc aaaatgtcaa attcaagtgc attatcagaa ggatgaagaa 360

aaagctgatc ctctttttaa tccggtaaaa atggggaaat gttcgtttaa cacattgaag 420

gttaaaaacg ctattctgga acgggccgga ggaaatattg aactgtatac ccaacgctat 480

caatcttcat ttcggaccct ggaaaatgtt ttaaatttct cacaatcgga gacatgtaag 540

actacagaaa agtctacgaa atgcacatta ccagaggctt taccgtctga acttaaggta 600

acgcctgaca atgtatcatt acctggtgcc tggagccttt cttccacgct gactgagata 660

tttctgttgc aagaggccca gggaatgcca caggtagcct gggggcgtat tacgggagaa 720

aaagaatgga gagatttgtt aagtctgcat aacgctcagt ttgatctttt gcaaagaact 780

ccagaagttg cccgtagtag agccacacca ttactcgata tgatagacac tacactattg 840

acaaatggtg caacagaaaa caggtatggc ataaaattac ctgtatctct gttgtttatt 900

gctggtcatg ataccaatct tgcaaattta agcggggctt tagatcttaa ctggtcgcta 960

cccggtcaac ccgataatac ccctcctggt ggggagcttg tattcgaaaa gtggaaaaga 1020

accagtgata atacggattg ggttcaggtt tcatttgttt atcagacgct gagagatatg 1080

agggatatac aaccgttgtc gttagaaaaa cctgccggca aagttgattt aaaattaatt 1140

gcatgtgaag agaaaaatat tcagggaatg tgttcgttaa aaagtttttc caggctcatt 1200

aaggaaattc gcgtgccaga gtgtgcagtt acggaa 1236

<210> 21
<211> 446
<212> PRT
<213> Buttiauxella sp.

<400> 21
Met Thr Ile Ser Ala Phe Asn Arg Lys Lys Leu Thr Leu His Pro Gly
1               5                   10                  15
Leu Phe Val Ala Leu Ser Ala Ile Phe Ser Leu Gly Ser Thr Ala Tyr
            20                  25                  30
Ala Asn Asp Thr Pro Ala Ser Gly Tyr Gln Val Glu Lys Val Val Ile
        35                  40                  45
Leu Ser Arg His Gly Val Arg Ala Pro Thr Lys Met Thr Gln Thr Met

50                  55                  60
Arg Asp Val Thr Pro Asn Thr Trp Pro Glu Trp Pro Val Lys Leu Gly
65                  70                  75                  80
Tyr Ile Thr Pro Arg Gly Glu His Leu Ile Ser Leu Met Gly Gly Phe
                85                  90                  95
Tyr Arg Gln Lys Phe Gln Gln Gln Gly Ile Leu Ser Gln Gly Ser Cys
            100                 105                 110
Pro Thr Pro Asn Ser Ile Tyr Val Trp Ala Asp Val Asp Gln Arg Thr
        115                 120                 125
Leu Lys Thr Gly Glu Ala Phe Leu Ala Gly Leu Ala Pro Glu Cys His
    130                 135                 140
Leu Thr Ile His His Gln Gln Asp Ile Lys Lys Ala Asp Pro Leu Phe
145                 150                 155                 160
His Pro Val Lys Ala Gly Thr Cys Ser Met Asp Lys Thr Gln Val Gln
                165                 170                 175
Gln Ala Val Glu Lys Glu Ala Gln Thr Pro Ile Asp Asn Leu Asn Gln
            180                 185                 190
His Tyr Ile Pro Phe Leu Ala Leu Met Asn Thr Thr Leu Asn Phe Ser
        195                 200                 205
Thr Ser Ala Trp Cys Gln Lys His Ser Ala Asp Lys Ser Cys Asp Leu
    210                 215                 220
Gly Leu Ser Met Pro Ser Lys Leu Ser Ile Lys Asp Asn Gly Asn Lys
225                 230                 235                 240
Val Ala Leu Asp Gly Ala Ile Gly Leu Ser Ser Thr Leu Ala Glu Ile
                245                 250                 255
Phe Leu Leu Glu Tyr Ala Gln Gly Met Pro Gln Ala Ala Trp Gly Asn
            260                 265                 270
Ile His Ser Glu Gln Glu Trp Ala Ser Leu Leu Lys Leu His Asn Val
        275                 280                 285
Gln Phe Asp Leu Met Ala Arg Thr Pro Tyr Ile Ala Arg His Asn Gly
    290                 295                 300
Thr Pro Leu Leu Gln Ala Ile Ser Asn Ala Leu Asn Pro Asn Ala Thr
305                 310                 315                 320
Glu Ser Lys Leu Pro Asp Ile Ser Pro Asp Asn Lys Ile Leu Phe Ile
                325                 330                 335
Ala Gly His Asp Thr Asn Ile Ala Asn Ile Ala Gly Met Leu Asn Met
            340                 345                 350
Arg Trp Thr Leu Pro Gly Gln Pro Asp Asn Thr Pro Pro Gly Gly Ala
        355                 360                 365
Leu Val Phe Glu Arg Leu Ala Asp Lys Ser Gly Lys Gln Tyr Val Ser
    370                 375                 380
Val Ser Met Val Tyr Gln Thr Leu Glu Gln Leu Arg Ser Gln Thr Pro
385                 390                 395                 400
Leu Ser Leu Asn Gln Pro Ala Gly Ser Val Gln Leu Lys Ile Pro Gly
                405                 410                 415
Cys Asn Asp Gln Thr Ala Glu Gly Tyr Cys Pro Leu Ser Thr Phe Thr
            420                 425                 430
Arg Val Val Ser Gln Ser Val Glu Pro Gly Cys Gln Leu Gln
        435                 440                 445

<210> 22
<211> 1338
<212> DNA
<213> Buttiauxella sp.


<400> 22
atgacgatct ctgcgtttaa ccgcaaaaaa ctgacgcttc accctggtct gttcgtagca      60

ctgagcgcca tattttcatt aggctctacg gcctatgcca acgacactcc cgcttcaggc     120

taccaggttg agaaagtggt aatactcagc cgccacgggg tgcgagcacc aaccaaaatg     180

acacagacca tgcgcgacgt aacacctaat acctggcccg aatggccagt aaaattgggt 240

tatatcacgc cacgcggtga gcatctgatt agcctgatgg gcgggtttta tcgccagaag 300

tttcaacaac agggcatttt atcgcagggc agttgcccca caccaaactc aatttatgtc 360

tgggcagacg ttgatcagcg cacgcttaaa actggcgaag ctttcctggc agggcttgct 420

ccggaatgtc atttaactat tcaccaccag caggacatca aaaaagccga tccgctgttc 480

catccggtga aagcgggcac ctgttcaatg gataaaactc aggtccaaca ggccgttgaa 540

aaagaagctc aaacccccat tgataatctg aatcagcact atattccctt tctggccttg 600

atgaatacga ccctcaactt ttcgacgtcg gcctggtgtc agaaacacag cgcggataaa 660

agctgtgatt tagggctatc catgccgagc aagctgtcga taaaagataa tggcaacaaa 720

gtcgctctcg acggggccat tggcctttcg tctacgcttg ctgaaatttt cctgctggaa 780

tatgcgcaag ggatgccgca agcggcgtgg gggaatattc attcagagca agagtgggcg 840

tcgctactga aactgcataa cgtccagttt gatttgatgg cacgcacgcc ttatatcgcc 900

agacataacg gcacgccttt attgcaggcc atcagcaacg cgctgaaccc gaatgccacc 960

gaaagcaaac tgcctgatat ctcacctgac aataagatcc tgtttattgc cggacacgat 1020

accaatattg ccaatatcgc aggcatgctc aacatgcgct ggacgctacc tgggcaaccc 1080

gataacaccc ctccgggcgg cgctttagtc tttgagcgtt tggccgataa gtcagggaaa 1140

caatatgtta gcgtgagcat ggtgtatcag actctcgagc agttgcgctc ccaaacacca 1200

cttagcctta atcaacctgc gggaagcgta cagctaaaaa ttcctggctg taacgatcag 1260

acggctgaag gatactgccc gctgtcgacg ttcactcgcg tggttagcca aagcgtggaa 1320

ccaggctgcc agctacag 1338

<210> 23
<211> 467
<212> PRT
<213> Aspergillus niger

<400> 23
Met Gly Val Ser Ala Val Leu Leu Pro Leu Tyr Leu Leu Ser Gly Val
1               5                   10                  15
Thr Ser Gly Leu Ala Val Pro Ala Ser Arg Asn Gln Ser Ser Cys Asp
            20                  25                  30
Thr Val Asp Gln Gly Tyr Gln Cys Phe Ser Glu Thr Ser His Leu Trp
        35                  40                  45
Gly Gln Tyr Ala Pro Phe Phe Ser Leu Ala Asn Glu Ser Val Ile Ser
    50                  55                  60
Pro Glu Val Pro Ala Gly Cys Arg Val Thr Phe Ala Gln Val Leu Ser
65                  70                  75                  80
Arg His Gly Ala Arg Tyr Pro Thr Asp Ser Lys Gly Lys Lys Tyr Ser
                85                  90                  95
Ala Leu Ile Glu Glu Ile Gln Gln Asn Ala Thr Thr Phe Asp Gly Lys
            100                 105                 110
Tyr Ala Phe Leu Lys Thr Tyr Asn Tyr Ser Leu Gly Ala Asp Asp Leu

115                 120                 125
Thr Pro Phe Gly Glu Gln Glu Leu Val Asn Ser Gly Ile Lys Phe Tyr
    130                 135                 140
Gln Arg Tyr Glu Ser Leu Thr Arg Asn Ile Val Pro Phe Ile Arg Ser
145                 150                 155                 160
Ser Gly Ser Ser Arg Val Ile Ala Ser Gly Lys Lys Phe Ile Glu Gly
                165                 170                 175
Phe Gln Ser Thr Lys Leu Lys Asp Pro Arg Ala Gln Pro Gly Gln Ser
            180                 185                 190
Ser Pro Lys Ile Asp Val Val Ile Ser Glu Ala Ser Ser Ser Asn Asn
        195                 200                 205
Thr Leu Asp Pro Gly Thr Cys Thr Val Phe Glu Asp Ser Glu Leu Ala
    210                 215                 220
Asp Thr Val Glu Ala Asn Phe Thr Ala Thr Phe Val Pro Ser Ile Arg
225                 230                 235                 240
Gln Arg Leu Glu Asn Asp Leu Ser Gly Val Thr Leu Thr Asp Thr Glu
                245                 250                 255
Val Thr Tyr Leu Met Asp Met Cys Ser Phe Asp Thr Ile Ser Thr Ser
            260                 265                 270
Thr Val Asp Thr Lys Leu Ser Pro Phe Cys Asp Leu Phe Thr His Asp
        275                 280                 285
Glu Trp Ile Asn Tyr Asp Tyr Leu Gln Ser Leu Lys Lys Tyr Tyr Gly
    290                 295                 300
His Gly Ala Gly Asn Pro Leu Gly Pro Thr Gln Gly Val Gly Tyr Ala
305                 310                 315                 320
Asn Glu Leu Ile Ala Arg Leu Thr His Ser Pro Val His Asp Asp Thr
                325                 330                 335
Ser Ser Asn His Thr Leu Asp Ser Ser Pro Ala Thr Phe Pro Leu Asn
            340                 345                 350
Ser Thr Leu Tyr Ala Asp Phe Ser His Asp Asn Gly Ile Ile Ser Ile
        355                 360                 365
Leu Phe Ala Leu Gly Leu Tyr Asn Gly Thr Lys Pro Leu Ser Thr Thr
    370                 375                 380
Thr Val Glu Asn Ile Thr Gln Thr Asp Gly Phe Ser Ser Ala Trp Thr
385                 390                 395                 400
Val Pro Phe Ala Ser Arg Leu Tyr Val Glu Met Met Gln Cys Gln Ala
                405                 410                 415
Glu Gln Glu Pro Leu Val Arg Val Leu Val Asn Asp Arg Val Val Pro
            420                 425                 430
Leu His Gly Cys Pro Val Asp Ala Leu Gly Arg Cys Thr Arg Asp Ser
        435                 440                 445
Phe Val Arg Gly Leu Ser Phe Ala Arg Ser Gly Gly Asp Trp Ala Glu
    450                 455                 460
Cys Phe Ala
465

<210> 24
<211> 1401
<212> DNA
<213> Aspergillus niger

<400> 24
atgggcgtct ctgctgttct acttcctttg tatctcctgt ctggagtcac ctccggactg      60

gcagtccccg cctcgagaaa tcaatccagt tgcgatacgg tcgatcaggg gtatcaatgc     120

ttctccgaga cttcgcatct ttggggtcaa tacgcaccgt tcttctctct ggcaaacgaa     180

tcggtcatct cccctgaggt gcccgccgga tgcagagtca ctttcgctca ggtcctctcc     240

cgtcatggag cgcggtatcc gaccgactcc aagggcaaga aatactccgc tctcattgag     300

gagatccagc agaacgcgac cacctttgac ggaaaatatg ccttcctgaa gacatacaac 360

tacagcttgg gtgcagatga cctgactccc ttcggagaac aggagctagt caactccggc 420

atcaagttct accagcggta cgaatcgctc acaaggaaca tcgttccatt catccgatcc 480

tctggctcca gccgcgtgat cgcctccggc aagaaattca tcgagggctt ccagagcacc 540

aagctgaagg atcctcgtgc ccagcccggc caatcgtcgc ccaagatcga cgtggtcatt 600

tccgaggcca gctcatccaa caacactctc gacccaggca cctgcactgt cttcgaagac 660

agcgaattgg ccgataccgt cgaagccaat ttcaccgcca cgttcgtccc ctccattcgt 720

caacgtctgg agaacgacct gtccggtgtg actctcacag acacagaagt gacctacctc 780

atggacatgt gctccttcga caccatctcc accagcaccg tcgacaccaa gctgtccccc 840

ttctgtgacc tgttcaccca tgacgaatgg atcaactacg actacctcca gtccttgaaa 900

aagtattacg gccatggtgc aggtaacccg ctcggcccga cccagggcgt cggctacgct 960

aacgagctca tcgcccgtct gacccactcg cctgtccacg atgacaccag ttccaaccac 1020

actttggact cgagcccggc taccttcccg ctcaactcta ctctctacgc ggacttttcg 1080

catgacaacg gcatcatctc cattctcttt gctttaggtc tgtacaacgg cactaagccg 1140

ctatctacca cgaccgtgga gaatatcacc cagacagatg gattctcgtc tgcttggacg 1200

gttccgtttg cttcgcgttt gtacgtcgag atgatgcagt gtcaggcgga gcaggagccg 1260

ctggtccgtg tcttggttaa tgatcgcgtt gtcccgctgc atgggtgtcc ggttgatgct 1320

ttggggagat gtacccggga tagctttgtg agggggttga gctttgctag atctgggggt 1380

gattgggcgg agtgttttgc t 1401

## Description

The present invention relates to the use of bacterial 3-phytases for the production of feed or food products. Further, the present invention relates to the use of bacterial 3-phytases for feeding animals and for the production of a food or feed additive.

Phytases are enzymes which can remove phosphate from organophosphate compounds such as phytate. Many types of phytases are known in the art and some of them are already used as an additive to animal feed products in order to improve metabolization and the utilization of phytate naturally present within animal feed stuff such as corn, wheat and soybeans. The classification of the phytases depends on the starting position of cleavage of phosphate from the phytate molecule in the first place. 3-phytases start with the phosphate on position 3 of the phytate molecule. 6-phytases start with the phosphate on position 6. Depending on the type of phytase, further cleavage of other positions occurs.

The addition of the phytases to the animals' feed enables better metabolization of the natural phytate content of the feed, as phosphate is an important element of an animal's diet, but also leads to a decrease of remaining phosphor in the animals' excrements. High phosphorous contents in animals' excrements usually leads to severe limitations of its use as manure as many countries set strict limitations.

In order to overcome these drawbacks of an insufficient natural phytate metabolization and a costly artificial phosphorous enrichment of feed which is also known to particularly increase remaining phosphorous content in manure, phytases have already been introduced in animal feed products to increase phytate metabolization in the animal's stomach and digestive tract.

Examples for such phytases which are currently commercially available originate from *Escherichia coli, Citrobacter braakii, Buttiauxella species and Aspergillus niger.*

Each of these phytase products has limitations in at least specific activity, dosage requirements, reaction time and accumulation of inhibiting intermediate compounds. First of its kind products contained fungal 3-phytases which require high enzyme dosing in order to achieve the desired effect. Currently available, commercial feed products try to overcome this effect by using 6-phytases of bacterial origin. A severe drawback of these 6-phytases is, however, an accumulation of the intermediate compound inositol-tetraphosphate which severly limits efficient phytate metabolization. In addition phytate and its intermediates, particularly inositol-tetraphosphate, form complexes with divalent ions such as Ca²⁺, Zn²⁺ and Mg²⁺. Further, many of the phytase products known within the art require the use of a high amount of phytase to guarantee a sufficient phosphate release regarding the actual critical retention time of the feed in the animals stomach of a maximum of 1.5 hours.

Therefore, there is a need within the state of the art to provide higher efficient ways to improve phytate metabolization when feeding animals. The inventors of the present invention have now surprisingly found that this need can be remedied by using bacterial 3-phytases.

Within a first aspect, the present invention is therefore directed to the use of at least one bacterial 3-phytase for the production of a feed or food product. Within a second aspect, the present invention is directed to the use of at least one bacterial 3-phytase for feeding animals. And in a final aspect the present aspect is directed to the use of at least one bacterial 3-phytase for the production of a food or feed additive.

Within the present application the term 3-phytase "is to be understood as any phytase falling in the class of E.C. 3.1.3.8 and are also referred to as: 1-phytase; myo-inositol-hexakisphosphate 3-phosphohydrolase; phytate 1-phosphatase or phytate 3 -phosphatase.

The term "bacterial phytase" within the present application is to be understood as comprising any phytase which is of bacterial origin. "Bacterial origin" pertains to any phytase of modified or non-modified form such as so-called wildtype-phytases isolated from any kind of bacterial genome but also to phytases in modified such as genetically modified or genetically engineered or mutated form.

According to the present invention phytases from the genus *Dickeya* and *Serratia* are particularly preferred, wherein phytases from *Dickeya sp., Serratia sp., Dickeya zeae, Dickeya chrysanthemi, Dickeya dadantii, Dickeya solani or Dickeya paradisiaca* are most preferred.

Within a particularly preferred embodiment phytases are used which are characterized by less than 25 % (wt./wt. free phosphate) accumulation of the intermediate product inositol-tetraphosphate after 35 % inorganic phosphate release within an assay of 2.7 mmol/L phytate at 37 °C and pH 5.5 using an enzyme dosage of 0.2 U/mL. Such phytases are particularly advantageous as an accumulation of the intermediate product inositol-tetraphosphate hampers full phosphate release of the phytate within the relevant residual time of the feed within the animals' stomach. Further, accumulation of the intermediate inositol-tetraphosphate hampers divalent ion utilization. Phytases commonly used for animal feed products accumulate huge amounts of this intermediate product prohibiting full phosphor release before the feed mass is leaving the animals' stomach for further digestion.

Less than 25 % (wt./wt.) accumulation of the intermediate product inositol-tetraphosphate is further achieved by implementing an extremely low enzyme dosage of 0.2 U/mL. Such phytases are therefore also highly advantageous for economical reasons. Particularly preferred is the use of phytases characterized by less than 20 % (wt./wt. free phosphate) accumulation or by less than 15 % (wt./wt. free phosphate) accumulation and most preferred of less than 10 % (wt./wt. free phosphate) accumulation of the intermediate product inositol-tetraphosphate after 35 % inorganic phosphate release within an assay of 2.7 mmol/L phytate at 37 °C and pH 5.5 using an enzyme dosage of 0.2 U/mL.

Within a further embodiment, phytases are used which are characterized by a phosphate release of at least 15 % from 2.7 mmol/L phytate at pH 5.5 after 1 hour using an enzyme concentration of 0.21 µg/mL. The use of these phytases is preferred because retention time of the feed within the stomach of the animal is usually around 1 hour. Therefore, phytases are preferred which release a majority of phosphor within the timeframe of 1 hour. The more phosphate can be released within the stomach retention time the less phytase can be used within the feed product. This will further contribute to economical advantages. It is thereby particularly preferred to use phytases characterized by a phosphate release of at least 20 %, even more preferred by at least 25 % also particularly preferred by at least 30 %, or by at least 35 % as well as by at least 40% or at least 45 % and most preferred by at least 50 % from 2.7 mmol/L phytate at pH 5.5 after 1 hour using an enzyme concentration of 0.21 µg/m L.

As temperature during digestion in the animal's stomach is greatly varying phytases with a temperature optimum of from 37 to 42 °C are used within a particularly preferred embodiment, wherein temperature optimum of from 35 to 45 °C are particularly preferred and from 30 to 50 °C are most preferred. In case the phytases of the present invention are used to feed fish, phytases with a temperature optimum of from 1 to 45 °C, preferably from 15 to 35 °C are used.

As pH within the animals' stomach is greatly varying due to the respective feed ratio consumed at a time within a particularly preferred embodiment phytases are used which show a pH optimum within a range of from pH 3.5 to 4.5, more preferred from pH 3.0 to 5.0, and most preferred from pH 2.5 to 5.5.

Within a further particularly preferred embodiment, bacterial 3-phytases with a sequence identity of at least 70 % to SEQ ID NO 1, preferably at least 75 % identity to SEQ ID NO 1, further preferred of at least 80 % identity to SEQ ID NO 1, particularly preferred of at least 85 % identity to SEQ ID NO1 also preferred of at least 90 % SEQ ID NO1, moreover particularly preferred of at least 95 % to SEQ ID NO 1 and most preferred of at least 99 % sequence identity to SEQ ID NO 1 are used. Particularly preferred bacterial 3-phytases are SEQ ID NO 3, SEQ ID NO 5, SEQ ID NO 7, SEQ ID NO 9, SEQ ID NO 11, SEQ ID NO 13 and SEQ ID NO 15.

The term "feed" product as used within the present application pertains to any product known to a person skilled in the art as suitable for feeding any kind of animal, preferably mammals and birds, such as but not limited to ruminants, pigs, deer, poultry but also comprises fish or other aquatic livestock generally referred to as seafood. Particularly preferred are feed products for non- ruminant animals, e.g. poultry, broilers, birds, chickens, turkeys, ducks, geese, and fowl; ruminant animals e.g. cows, cattle, horses, and sheep; pigs, swine, piglets, growing pigs, and sows; companion animals including but not limited to: cats, dogs, rodents, and rabbits; fish including but not limited to salmon, trout, tilapia, catfish and carp; and crustaceans including but not limited to shrimp and prawn.

The term "food" product as used within the present application pertains to any product known to a person skilled in the art as suitable human consumption.

The feed or food product of the present invention preferably contains starch. The starch-containing feed components typically include vegetable material such as cereal(s), e.g., one or more of corn (maize), wheat, barley, rye, rice, sorghum and millet, and/or tubers such as potatoes, cassava and sweet potato. The vegetable material may be milled, e.g., wet or dry milled grain, or distillers dry grain solids.

The feed or food product of the present invention preferably contains protein-rich feed ingredients such as soybean (preferably soybean meal), rapeseed, palm kernel, cotton seed and sunflower.

Within a further embodiment, the feed or food product comprises at least one compound selected from the group consisting of vitamins, minerals, organic acids, probiotic components, oils, fats, pigments, growth factors and antimicrobial agents.

Within a further embodiment, the feed or food product comprises at least one one or more enzymes in addition to the bacterial 3-phytase, particularly feed enzymes which improve the digestibility of the feed, e.g. another phytase, an amylase or a protease, aminopeptidase, carbohydrase, carboxypeptidase, catalase, cellulase, chitinase, cutinase, cyclodextrin glycosyltransferase, deoxyribonuclease, esterase, alpha- galactosidase, beta-galactosidase, glucoamylase, alpha-glucosidase, beta-glucosidase, haloperoxidase, invertase, laccase, lipase, mannosidase, oxidase, pectinolytic enzyme, pepti- doglutaminase, peroxidase, polyphenoloxidase, proteolytic enzyme, ribonuclease, transglutaminase, or xylanase. The feed enzyme(s) may be derived from microorganisms such as bacteria or fungi or from plants or animals.

Within another preferred embodiment the feed or food product is in granulate, compactate, extrudate or liquid form.

Another aspect of the present invention pertains to the use of bacterial 3-phytases for feeding animals. Thus, the bacterial 3-phytases are not incorporated into a separate product but directly given to the animals.

If the bacterial 3-phytase is directly given to the animal, the phytase is preferably provided in the form of an extrudate or granulate, particularly a coated granulate, e.g. with a coating comprising a salt, e.g. at least 60 % w/w of the salt. The salt may be selected from the group consisting of NaCl, KCI, Na₂SO₄, K₂SO₄ and MgSO₄.

Another aspect of the present invention pertains to the use of bacterial 3-phytases for the production of a food or feed additive. A food or feed additive is a product which can be incorporated into a commercial food product such as pelleted or extrudated mixtures of grains, corn, hay, molasses and straw and may be in dry or liquid form. 3-phytases as defined before for use for the production of a feed or food product are particularly preferred.

As used herein "liquid form" pertains to a composition preferably also comprising a buffer, a stabilizer, and an anti-microbial agent and may even comprise at least one further enzyme, wherein the enzyme is an amylase, a cellulase, a lactase, a lipase, a protease, a catalase, a xylanase, a beta- glucanase, a mannanase, an amylase, an amidase, an epoxide hydrolase, an esterase, a phospholipase, a transaminase, an amine oxidase, a cellobiohydrolase, an ammonia lyase, or any combination thereof.

As used herein "dry form" pertains to a composition preferably also comprising one or more of the following components: a carrier, a buffer, a stabilizer, a binding agent, a plasticizer, an anti-microbial agent. Particularly preferred are dry forms comprising sucrose, sodium chloride, sorbitol, sodium citrate, potassium sorbate, sodium benzoate, sodium propionate, guar gum and/or wheat flour.

### Figures and examples

The present invention is in the following further described by examples and figures. It is emphasized that the examples and figures have only exemplary character and do not limit the scope of the present invention.
- Figure 1: shows a comparison between bacterial 3-phytases according to the present invention and bacterial 6-phytases currently used within the state of the art for feeding animals with respect to accumulation of the intermediate product inositol-tetraphosphate.
- Figure 2: shows a comparison of bacterial 3-phytases according to the present invention and fungal 3-phytases which are also known within the state of the art to be used for feeding animals and have been used in the past relating to a phosphate release pertaining to the same amount of phytase used within the assay.
- Figure 3: shows a comparison of bacterial 3-phytases according to the present invention and bacterial 6-phytases currently used within the state of the art for feeding animals with respect to phosphate release from the intermediate product inositol-tetraphosphate

### Methods

The following methods have been applied within the examples:

### State of the art phytases

The following phytases have been tested within the examples:

| Short name | Species | SEQ ID NO |
|---|---|---|
| E.coli | *Escherichia coli* | 17 |
| Cit.braaki | *Citrobacter braakii* | 19 |
| Butt.sp | *Buttiauxella sp.* | 21 |
| A.niger | *Aspergillus niger* | 23 |

### Determination of enzyme concentrations

SEQ ID NO 1 concentration was determined via UV absorbance using its molar extinction coefficient. All other phytase solutions and extracts were quantified via an in house SDS gel quantification method using a SEQ ID NO 1 calibration curve. Phytase samples were applied to a SDS gel which was subsequently stained with Sypro Ruby (Thermo Fisher: S12000). The gel image was recorded on a standard Bio-Rad gel documentation instrument. Image analysis was performed using ImageLab software (Bio-Rad). Protein concentration was determined by signal integration of the phytase specific SDS gel bands using a SEQ ID NO 1 calibration curve on the same SDS gel.

### Determination of phytase activity

5 µL enzyme solution (in 100 mmol/L sodium acetate buffer, pH 5.5, 0.05 % (w/v) Triton X-100) were incubated with 95 µL 2,88 mmol/L sodium phytate (Sigma 68388, lot BCBM4006V) in 100 mmol/L sodium acetate buffer, pH 5.5 at 37 °C for 15 min. The reaction was stopped by adding 100 µL 10 % (w/v) trichloroacetic acid. Subsequently, 100 µL of the stopped enzymatic reaction was mixed with 100 µL molybdate reagent (aqueous solution of 1.2 % (w/v) ammonium molybdate; 4.4 % (v/v) sulfuric acid, and 27 mg/mL (w/v) ferrous sulfate) and the solution was incubated for 15 min at room temperature. Absorbance at 700 nm was determined and the amount of released inorganic phosphate was calculated using inorganic phosphate standard solutions (0 -1.5 mmol/L). One unit of phytase activity was defined as the amount of enzyme that releases 1 µmol phosphate per min at 37 °C.

### Determination of phytate content in the commercial phytate preparation

The standard substance phytic acid sodium salt hydrate (Sigma Art. 68388, lot BCBM4006V) contains phytate, sodium and water. The phytate content is not available by a certificate of analysis and was determined by acid hydrolysis and subsequent determination of free phosphorus by ICP-OES. 200 mg phytate sodium salt hydrate (Sigma Art. 68388, lot BCBM4006V) was weighted in a teflon tube. Subsequently, 1 mL water, 1 mL sulfuric acid 98 %, and 4 mL hydrogen peroxide solution 30 % were added. The tube was closed with the lid and bursting cap and incubated at 300 °C for 1 h in an autoclave. The hydrolysate was transferred quantitatively to a 50 mL volumetric flask and fill up to 50 mL with water. This solution was analysed for the content of phosphorus by ICP-OES according DIN EN ISO 11885 E22. The content of phytate was calculated by the weight, the content of phosphorus and molar masses. The content of free phytate in phytic acid sodium salt hydrate (Sigma Art. 68388, lot BCBM4006V) was 54.7 %.

### Determination of different inositol-phosphate species by HPAEC-UV

The determination of the different inositol-phosphate species was performed by high performance anion exchange chromatography with post column derivatisation using an UV detector at 290 nm (HPAEC-UV). The chromatographic system was a Dionex ICS-3000 with dual pump (IC-3000 DP), dual column oven (ICS-3000 DC), cooled autosampler (ICS-3000 AS) and UV detector (ThermoFisher MWD-3000). Instead of a post column delivery system a knitted reaction coil (4 mm system, 375 µL) was used for mixing the flows of both pump systems to perform the derivatisation reaction before detection. System 1 was used to separate the different inositol-phosphate species by using a Dionex CarboPac PA100 Guard column 4 x 50 mm and a Dionex CarboPac PA100 analytical column 4 x 250 mm. For gradient elution, HPLC grade water (pump system 1/canal A) and 0.5 M hydrochloric acid (pump system 1/canal B) were used at a flow rate of 1.0 mL/min. The gradient conditions were as follows: 0 min. 5 % B; 0-8 min. 5-10 % B; 8-25 min. 10-35 % B; 25-35 min. 35-100 % B; 35-42 min. 100 % B; 42-43 min 100-5 % B; 43-55 min 5 % B. Pump system 2 provides the post column reaction solution (0.33 M perchloric acid with 0.1 % Fe(NO₃)₃) to a tee connector in combination with the knitting coil at a flow rate of 0.4 mL/min. The injection volume was about 50 µL at a column temperature of 30 °C and an autosampler temperature of 10 °C. The run time was 55 min. The signals were detected at 290 nm and manually intregrated. The standard stock solution was prepared as follows: 100 mg phytic acid sodium salt hydrate (Sigma Art. 68388, lot BCBM4006V) was diluted in 100 mL Phosphate Standard Solution 1000 mg/L (Merck Art. 119898). The dilutions to several calibration levels were performed with water (HPLC grade) to end up with the following concentrations: 0.1 mg/mL Phytic acid sodium salt hydrate/Phosphate, 0.2 mg/mL Phytic acid sodium salt hydrate/Phosphate, 0.4 mg/mL Phytic acid sodium salt hydrate/Phosphate, 0.6 mg/mL Phytic acid sodium salt hydrate/Phosphate, and 0.8 mg/mL Phytic acid sodium salt hydrate/Phosphate.

**List of Retention Times of the inositol-phosphate species:**

| Substance | Retention time [min] |
|---|---|
| Phsophate | 3.45 |
| 1,4-Inositol-diphosphate | 11.81 |
| 4,5-Inositol-diphosphate | 12.33 |
| 1,2,3-Inositol-triphosphate | 17.86 |
| 1,4,5-Inositol-triphosphate | 18.27 |
| 1,5,6-Inositol-triphosphate | 18.69 |
| 1,2,4,5-1 nositol-tetraphosphate | 24.38 |
| 1,3,4,5-1 nositol-tetraphosphate | 25.39 |
| 2,4,5,6-1 nositol-tetraphosphate | 27.63 |
| 1,4,5,6-1 nositol-tetraphosphate | 28.82 |
| 1,2,3,4,5-Inositol-pentaphosphate | 32.12 |
| 1,2,4,5,6-Inositol-pentaphosphate | 34.40 |
| 1,2,3,4,5,6-Inositol-hexaphosphate (Phytate) | 39.07 |

### Examples

### Example 1 Determination of phytate degradation pattern

Enzymatic reactions were performed by incubating a 2.7 mmol/L phytate solution (Sigma 68388, lot BCBM4006V) in 100 mmol/L sodium acetate buffer, pH 5.5 at 37 °C with 0.2 U/mL of phytase. Time resolved enzymatic phytate degradation data was recorded by stopping the enzymatic reaction at different time points via immediate incubation at 99 °C for 10 min in a thermo shaker. The samples were analyzed according to method "Determination of different inositol-phosphate species by HPAEC-UV". Peak areas of inositol-phosphate isomers (inositol-phosphates with same amount of phosphate residues) were summed up. Subsequently, peak areas were corrected by normalization of the signals to the amount of phosphate residues of the different inositol-phosphate species (i.e. phytate contains six phosphate residues resulting in a six times higher detector response factor than the one of inorganic phosphate). For comparison of the degradation pattern, data of kinetic time points were taken at which equal amounts of phosphate had been released.

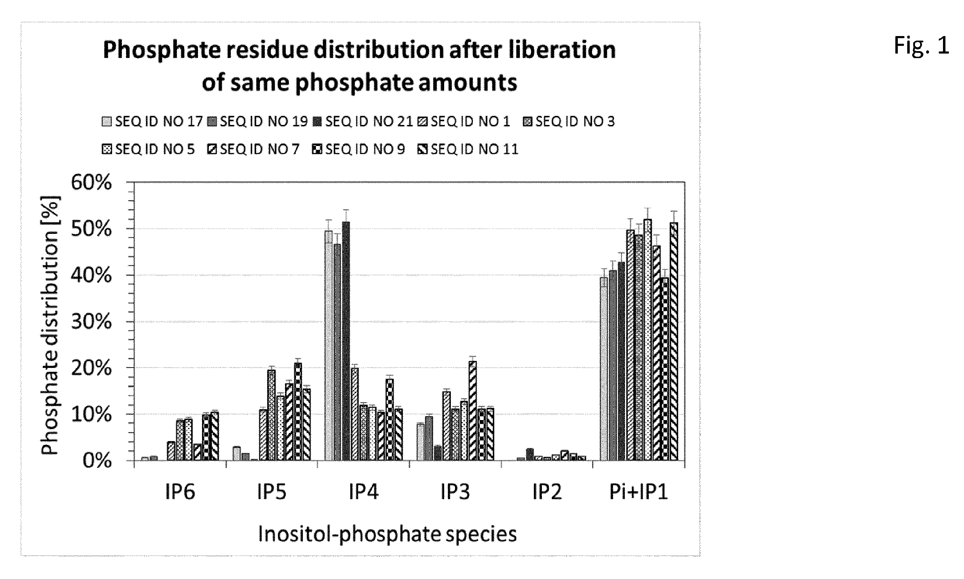

The results are shown in Fig. 1.

### Example 2 Determination of phosphate release data at same enzyme amounts of phytase

5 µL of enzyme solution (in 100 mmol/L sodium acetate buffer, pH 5.5, 0.05 % (w/v) Triton X-100) are incubated with 95 µL 2.88 mmol/L sodium phytate (Sigma 68388, lot BCBM4006V) in 100 mmol/L sodium acetate buffer, pH 5.5 at 37 °C for 1 h. The final enzyme concentration in the reaction is 0.21 µg/mL. The reaction is stopped by adding 100 µL 10 % (w/v) trichloroacetic acid. Subsequently, the stopped enzymatic reaction is diluted 1/10 and 100 µL of the dilution is mixed with 100 µL molybdate reagent (aqueous solution of 1.2 % (w/v) ammonium molybdate; 4.4 % (v/v) sulfuric acid, and 27 mg/mL (w/v) ferrous sulfate). The solution is incubated for 15 min at room temperature. Absorbance at 700 nm is determined and the amount of released inorganic phosphate is calculated using inorganic phosphate standard solutions (0 -1.5 mmol/L).

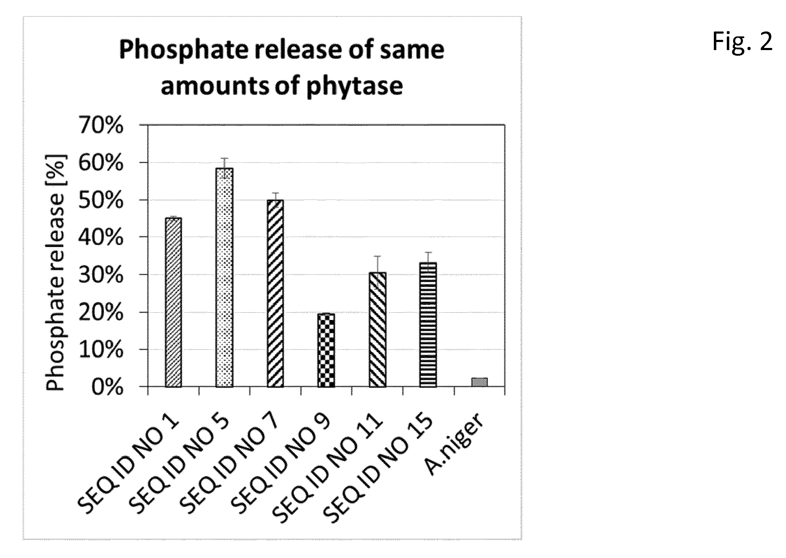

The results are shown in Fig. 2.

### Example 3 Determination of phosphate release from inositol-tetraphosphate

An Inositol-tetraphosphate preparation was produced by incubation of 2.7 mmol/L phytate in 100 mmol/L sodium acetate buffer, pH 5.5 at 37 °C for 1 h with 0.1 U/mL of SEQ ID NO 17. After the reaction, the enzyme was inactived by incubation at 95 °C for 30 min. The resulting 2.7 mmol/L inositol-tetraphosphate solution was used as a substrate in an enzymatic reaction according to Example 2 with a final phytase concentration of 0.2 U/mL.

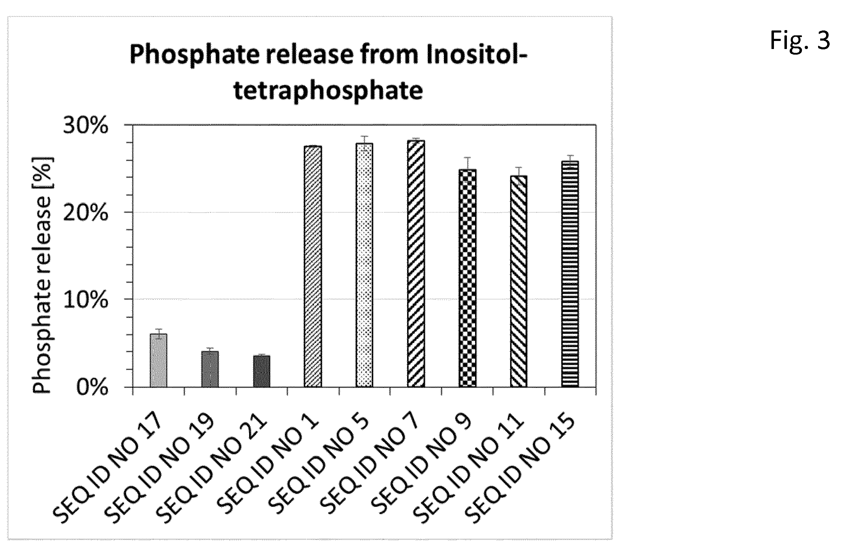

The results are shown in Fig. 3. The abbreviations used in the figure are: IP6: Inositol-hexaphosphate; IP5: Inositol-pentaphosphate; IP4: Inositol-tetraphosphate; IP3: Inositol-triphosphate; IP2: Inositol-diphosphate; IP1: Inositol-monophosphate; Pi: phosphate.

## Claims

1. Use of at least one bacterial 3-phytase for the production of a feed or food product.

2. Use according to claim 1, wherein the phytase is **characterized by** less than 25 % (wt./wt. free phosphate) accumulation of the intermediate product inositol-tetraphosphate after 35 % inorganic phosphate release within an assay of 2.7 mmol/L phytate at 37 °C and pH 5.5 using an enzyme dosage of 0.2 U/mL.

3. Use according to claim 1 or 2, wherein the phytase is **characterized by** a phosphate release of at least 15 % from 2.7 mmol/L phytate at pH 5.5 after 1 hour using an enzyme concentration of 0.21 µg/mL.

4. Use according to any of the foregoing claims, wherein the phytase is a phytase from the genus *Dickeya* or *Serratia.*

5. Use according to claim 4, wherein the phytase is a phytase from *Dickeya sp., Serratia sp., Dickeya zeae, Dickeya chrysanthemi, Dickeya dadantii, Dickeya soloni or Dickeya paradisiaca.*

6. Use according to any of the foregoing claims, wherein the phytase shows at least 70 % sequence identity to SEQ ID NO 1.

7. Use according to any of the foregoing claims, wherein the phytase shows a temperature optimum of from 30 to 65 °C and/or a pH optimum of from pH 2.5 to 5.5.

8. Use according to any of the foregoing claims, wherein the feed or food product further comprises at least one compound selected from the group consisting of vitamins, minerals, organic acids, probiotic components, oils, fats, pigments, growth factors, antimicrobial agents, cellulose, starch.

9. Use according to any of the foregoing claims, wherein the feed or food product is in dry or liquid form.

10. Use of a bacterial 3- phytase for feeding animals.

11. Use of a bacterial 3- phytase for the production of a food or feed additive.
